# EUROPÄISCHE PATENTSCHRIFT

(11) **EP 2 073 136 B1**
(45) Veröffentlichungstag und Bekanntmachung des Hinweises auf die Patenterteilung: **25.10.2017**
(21) Anmeldenummer: 07124033.7
(22) Anmeldetag: 21.12.2007
(51) Int. Cl.: G06Q 50/00, G06F 19/00, G06F 17/30, G06Q 50/22

(54) **System und Verfahren zum Erzeugen von Auswertedaten**
System and method for producing evaluation data
Système et procédé destinés à la production de données d'évaluation

(43) Veröffentlichungstag der Anmeldung: 24.06.2009
(73) Patentinhaber: Agfa HealthCare GmbH, 53227 Bonn (DE)
(72) Erfinder: Schilken, Jörg, 54329 Konz (DE)
(74) Vertreter: Linsmeier, Josef

(56) Entgegenhaltungen:
- EP-A- 1 739 605
- WO-A-2006/081041
- WO-A-2007/052213
- DE-A1- 4 440 178
- DE-A1-102005 005 243
- DE-A1-102006 008 507
- US-A- 5 077 666
- US-A1- 2001 049 610
- DON CHAMBERLIN: "Using the new DB2, IBM's Object-Relational Database System" 1996, MORGAN KAUFMANN PUBLISHERS, INC , SAN FRANSISCO, CA, USA , XP002504698 * Seite 455 - Seite 465 *

## Beschreibung

Die vorliegende Erfindung betrifft ein System und ein Verfahren zum Erzeugen von Auswertedaten aus Ausgangsdaten, die in einer Datenbank abgespeichert sind.

In einer Datenbank, die üblicherweise in einem Rechnernetzwerk mit einer Vielzahl von miteinander verbundenen Rechnern angeordnet ist, können Ausgangsdaten gespeichert werden, die von verschiedenen, auf den Rechnern ausgeführten, Applikationen erzeugt und/oder über Schnittstellen empfangen und in die Datenbank geschrieben werden. Als Ausgangsdaten werden in der Regel sowohl Stamm- als auch Bewegungsdaten in der Datenbank abgespeichert. Stammdaten werden auch als Grund- oder Referenzdaten bezeichnet und werden relativ selten geändert. Sie können beispielsweise eine Organisationsstruktur beschreiben. Bewegungsdaten werden z.B. bei unterschiedlichen Prozessen innerhalb einer Organisationsstruktur und damit in der Regel häufiger als Stammdaten geändert.
Eine solche Datenbank wird beispielsweise in einem Rechnernetzwerk in einer Krankenhausumgebung eingesetzt. Die auf den verschiedenen Rechnern des Rechnernetzwerks laufenden Applikationen beinhalten beispielsweise ein Modul für die medizinische Pflege, mit dem z.B. das Verabreichen von Medikamenten, Verbandsmaterialien usw. erfasst werden kann, ein Modul für das Rechnungswesen, mit dem z.B. der Materialverbrauch des Krankenhauses erfasst werden kann, oder ein Modul für die Logistik, d.h. insbesondere für den Warenein- und Warenausgang. Die Datenbank ist insbesondere eine relationale Datenbank, die z.B. Namens-, Fall- und Medikamententabellen usw. enthält.
In der Datenbank abgespeicherte Stammdaten können insbesondere die Organisationsstruktur des Krankenhauses mit seinen verschiedenen Stationen, Abteilungen, Räumen und Ambulanzen usw. abbilden. Bewegungsdaten geben beispielsweise die Neuaufnahme oder die Entlassung eines Patienten, die Verlegung eines Patienten von einer Station auf eine andere Station, die Verabreichung von Medikamenten an Patienten usw. an.

Aus den in der Datenbank abgespeicherten Ausgangsdaten können mittels eines Auswerteprogramms Auswertedaten erzeugt werden, die Kennzahlen der unterschiedlichen Prozesse innerhalb des Krankenhauses umfassen und damit z.B. über die jeweils aktuelle Anzahl von im Krankenhaus behandelten Patienten Aufschluss geben. Je nach Art der jeweils gewünschten Kennzahl müssen zum Erzeugen der Auswertedaten komplizierte Berechnungen durchgeführt und die Ausgangsdaten zusammengefasst und interpretiert werden. Dies ist mitunter sehr zeitintensiv.
Die erzeugten Auswertedaten werden visualisiert, indem sie z.B. auf einem Monitor in Textform und/oder graphischer Form, dargestellt und/oder auf Papier ausgegeben werden. Es ist auch möglich, die Auswertedaten in Dateiform auszugeben. Dies kann insbesondere in einem sogenannten CSV- oder einem XML-Format geschehen.

Eine Auswertung der in der Datenbank abgespeicherten Ausgangsdaten wird üblicherweise dann durchgeführt, wenn die Auswertedaten von einem Benutzer angefordert werden. Dieser muss dann die für die Auswertung benötigte Zeit abwarten, bis die bei der Auswertung erzeugten Auswertedaten tatsächlich vorliegen und ausgegeben werden können. Dies kann unter Umständen unerwünscht lange dauern. Darüber hinaus wird die Rechenkapazität des Rechners, der die Auswertungen durchführt, während der Auswertung stark beansprucht sowie das gesamte Rechnernetzwerk wegen der mit der Auswertung verbundenen Datenübertragung zwischen der Datenbank und dem Rechner stark belastet.

Nach der Anforderung von Auswertedaten durch den Benutzer steht der Rechner dem Benutzer daher für weitere Arbeiten nur sehr begrenzt zur Verfügung. Darüber hinaus verlangsamt sich mit der Anzahl der gestarteten Auswertungen die Datenübertragungsrate des Rechnernetzwerks.

Dokument EP-A-1 739 605 wird als nächstliegender Stand der Technik gegenüber dem Gegenstand des Anspruchs 1 angesehen. Es offenbart eine klinische Kommunikationseinheit und ein Krankenhausinformationssystem zum Informationsaustausch, wobei elektronische medizinische Akten mit Ereignisinformationen bezüglich Patienten und Ärzten verknüpft werden. Eine Extraktionseinheit für elektronische medizinische Akten extrahiert Informationen zu einer medizinischen Akte aus einer Datenbank und sendet diese zu einer Informationsaktualisierungseinheit und/oder einer Anzeigekontrolleinheit. Eine Extraktionseinheit für klinische Kommunikationsinformationen extrahiert Informationen zur klinischen Kommunikation aus einer klinischen Kommunikationsdatenbank und sendet diese zur Informationsaktualisierungseinheit und/oder Anzeigekontrolleinheit. Die Anzeigekontrolleinheit zeigt die elektronische medizinische Information und die klinische Kommunikationsinformation, während sie diese miteinander verknüpft. Die klinischen Kommunikationsinformationen beinhalten zumindest Ereignisinformationen für den Patienten, Ereignisinformationen für medizinische Versorgungskräfte und Nachrichten an/von medizinische/n Versorgungskräfte/n. Vorzugsweise beinhalten die Informationen zur elektronische medizinischen Akte Informationen zur Diagnose und Order-Informationen. Die Anzeigekontrolleinheit erzeugt eine Planungstabelle, welche die Ereignisinformationen für die Patienten und/oder die Ereignisinformationen für die medizinischen Versorgungskräften beinhaltet, und zeigt die Diagnose- und Behandlungsakten, die Order-Informationen, die Verlaufsuntersuchungsformulare und/oder die klinischen Wege aus der Planungstabelle an. Vorzugsweise gibt die Anzeigekontrolleinheit eine Warnung aus, wenn eine geplante Zeit, um ein Ereignis für die Patienten und/oder ein Ereignis für die medizinischen Versorgungskräfte umzusetzen, abgelaufen ist.

Es ist die Aufgabe der Erfindung, auf der Grundlage aktueller Ausgangsdaten erzeugte Auswertedaten auf technisch einfache Weise schnell und zuverlässig zur Verfügung stellen zu können.

Diese Aufgabe wird gemäß der technischen Lehre der Ansprüche 1, 11 und 14 gelöst.

Das erfindungsgemäße System zum Erzeugen von Auswertedaten umfasst eine Datenbank zum Abspeichern von Ausgangsdaten, einen Speicher zum Abspeichern eines Auswerteprogramms zum Auswerten von in der Datenbank abgespeicherten Ausgangsdaten sowie eine Steuerung, wobei bei Eintreten eines vorgegebenen Ereignisses eine Auswerteaufforderung zum Auswerten von Ausgangsdaten erzeugt wird, von der Steuerung überprüft wird, ob eine Auswerteaufforderung erzeugt worden ist, und im Falle des Vorhandenseins einer Auswerteaufforderung das in dem Speicher abgespeicherte Auswerteprogramm in Abhängigkeit von der Auswerteaufforderung gestartet wird und das Auswerteprogramm in Abhängigkeit von der Auswerteaufforderung gestartet wird, welches in Abhängigkeit von der Auswerteaufforderung auf in der Datenbank abgespeicherte Ausgangsdaten zugreift und daraus die Auswertedaten erzeugt.
Aufgrund der Erfindung kann das Eintreten vorgegebener Ereignisse, die eine Änderung von Ausgangsdaten zur Folge haben, kontinuierlich überwacht werden, sodass die Auswertedaten laufend an die - mit den jeweiligen Ereignissen einhergehenden - Änderungen der Ausgangsdaten angepasst, d.h. entsprechend aktualisiert, werden können. Die Auswertedaten stehen daher jederzeit aktualisiert zur Verfügung. Dies hat den Vorteil, dass der Benutzer nach dem Anfordern der Auswertedaten nicht mehr zu warten braucht, bis die Auswertedaten aus den - zumindest teilweise geänderten - Ausgangsdaten ermittelt worden sind. Ein weiterer Vorteil der Erfindung liegt auch darin, dass die Rechenkapazität des Rechners, der die Auswertungen durchführt, sowie die Übertragungskapazität des Rechnernetzwerks nach dem Anfordern der Auswertedaten nicht mehr durch die Berechnungen der Auswertedaten beansprucht werden. Unmittelbar nach einer Anforderung von Auswertedaten steht daher der Rechner dem Benutzer sofort für weitere Arbeiten bei gleichzeitig geringerer Beanspruchung des Rechnernetzwerks zur Verfügung.
Zusammenfassend ist festzuhalten, dass durch die Erfindung die aus den Ausgangsdaten erzeugten Auswertedaten auf technisch einfache Weise schnell und zuverlässig zur Verfügung gestellt werden können.
In einer vorteilhaften Ausgestaltung der Erfindung ist die Steuerung so ausgelegt, dass das Vorhandensein einer Auswerteaufforderung kontinuierlich, d.h. ohne Unterbrechung, überprüft wird. Im Sinne der Erfindung ist "kontinuierlich" so zu verstehen, dass das Vorhandensein einer Auswerteaufforderung in kürzesten technisch möglichen Zeitabständen erfolgt. Dadurch wird erreicht, dass das in dem Speicher abgespeicherte Auswerteprogramm praktisch unmittelbar nach dem Erzeugen der Auswerteaufforderung gestartet werden kann, so dass eine sehr schnelle Aktualisierung der Auswertedaten erfolgen kann. Die aktualisierten Auswertedaten stehen dadurch bereits in kürzester Zeit nach dem Eintreten des vorgegebenen Ereignisses, z.B. einer Änderung von Ausgangsdaten, zur Verfügung.

Alternativ kann die Steuerung so konfiguriert sein, dass das Vorhandensein einer Auswerteaufforderung in festlegbaren längeren Zeitabständen, z.B. alle 10 Sekunden, erfolgt.

In einer weiteren vorteilhaften Ausgestaltung der Erfindung weist das Auswerteprogramm mehrere unterschiedliche Programmteile zum Auswerten unterschiedlicher Ausgangsdaten auf, wobei in Abhängigkeit von dem vorgegebenen Ereignis unterschiedliche Typen von Auswerteaufforderungen erzeugt werden und die Steuerung so ausgestaltet ist, dass sie in Abhängigkeit von dem jeweils erzeugten Typ der Auswerteaufforderung einen der Programmteile startet. Bei dieser Ausgestaltung umfasst das Auswerteprogramm mehrere Programmteile, vorzugsweise sog. Plugins, welche unterschiedlichen Typen der Auswerteaufforderung zugeordnet sind, d.h. ein bestimmter Programmteil wird nur bei Vorliegen eines bestimmten Typs von Auswerteaufforderungen aufgerufen. Man spricht in diesem Zusammenhang auch davon, dass dieser Programmteil einen bestimmten Typ der Auswerteaufforderung "abonniert" hat. Bei dieser Ausgestaltung der Erfindung kann das Erzeugen der Auswertedaten besonders effizient erfolgen, da nur diejenigen Auswertedaten aufgrund des eingetretenen vorgegebenen Ereignisses neu berechnet werden, für die dies erforderlich ist, um sie an das vorgegebene Ereignis anzupassen, d.h. zu aktualisieren.
Im Sinne der Erfindung kann eine Auswerteaufforderung auch als "Event" und ein bestimmter Typ der Auswerteaufforderung als "Eventtyp" bezeichnet werden.

In einer weiteren vorteilhaften Ausgestaltung ist die Steuerung so ausgestaltet, dass in Abhängigkeit von dem jeweils gestarteten Programmteil auf in der Datenbank abgespeicherte Ausgangsdaten zugegriffen wird. Dies gewährleistet ein noch effizienteres Erzeugen der Auswertedaten. Es kann dabei davon ausgegangen werden, dass sich bei Eintreten des vorgegebenen Ereignisses nicht alle, sondern nur bestimmte, für das Erzeugen der Auswertedaten relevante Ausgangsdaten geändert haben. Durch den Programmteil ist vorteilhafterweise festgelegt, auf welche Ausgangsdaten zugriffen wird und welche Auswertedaten erzeugt werden. Es ist somit durch den Programmteil insbesondere eine Zuordnung von Ausgangsdaten zu den jeweils zu erzeugenden Auswertedaten festgelegt.
Bei einer Weiterbildung der o.g. Ausgestaltungen werden bei einem zwei- oder mehrmaligen Eintreten von vorgegebenen Ereignissen im Zusammenhang mit einem bestimmten Auswertungsobjekt, z.B. eines Falles oder Patienten, zwei bzw. mehrere Auswerteaufforderungen erzeugt. Die Steuerung ist hierbei so ausgestaltet, dass nur für eine dieser Auswerteaufforderungen einer der Programmteile gestartet wird, wenn die übrigen Auswerteaufforderungen ein Starten des selben Programmteils zur Folge hätten. Der Status der übrigen Ausgabeaufforderungen wird hierbei vorzugsweise auf "nicht bearbeiten" gesetzt.
Die zwei bzw. mehreren Auswerteaufforderungen können gleichen oder unterschiedlichen Typs sein. Entscheidend bei dieser Ausgestaltung ist, dass die Auswerteaufforderungen - egal welchen Typs diese sind -jeweils denselben Programmteil starten würden; welcher dadurch zwei- bzw. mehrmals ausgeführt werden würde. Die Steuerung ist hierbei so konfiguriert, dass sie dies erkennt und diesen Programmteil nur einmal ausführt. Dies wird anhand der folgenden beiden Beispiele veranschaulicht.

Beispiel 1: Werden z.B. aufgrund einer kurz aufeinander folgenden zwei- oder mehrmaligen Verlegung eines Falles, der in diesem Beispiel das Auswertungsobjekt darstellt, im Krankenhaus und der damit verbundenen Änderungen von Ausgangsdaten zwei bzw. mehrere Auswerteaufforderungen eines bestimmten Typs erzeugt, so wird dies von der Steuerung erkannt, und es wird der diesem Typ der Auswerteaufforderung zugeordnete Programmteil nicht für jede Verlegung, d.h. zwei- bzw. mehrmals, sondern nur für eine Verlegung, d.h. einmal, gestartet.
Beispiel 2: Werden z.B. für eine Person, welche hier das Auswertungsobjekt darstellt, sukzessive Informationen über Name, Geschlecht, Alter etc. eingegeben oder geändert und hierfür auch entsprechende Auswerteaufforderungen unterschiedlichen Typs erzeugt, so wird bei einer zwei- oder mehrfachen Änderung dieser Daten der den Auswerteaufforderungen dieser Typen jeweils zugeordnete selbe Programmteil nur einmalig ausgeführt. Die übrigen zu demselben Auswerteobjekt, d.h. diesem Patienten, gehörenden Auswerteaufforderungen werden deaktiviert, so dass hierzu keine Programmteile des Auswerteprogramms ausgeführt werden.

Durch diese Weiterbildung der Erfindung wird erreicht, dass Änderungen in den Ausgangsdaten einerseits so schnell und so oft wie nötig und andererseits so selten wie möglich bei der Ermittlung der Auswertedaten berücksichtigt werden. Die Belastung der Kapazität des Systems, d.h. der beteiligten Rechner, der Datenbank sowie des Rechnernetzwerks, wird dadurch niedrig gehalten.
In einer bevorzugten Ausgestaltung der Erfindung ist das vorgegebene Ereignis eine Änderung von Ausgangsdaten in der Datenbank. Das Erkennen der Änderung von Ausgangsdaten erfolgt vorzugsweise automatisch, beispielsweise mittels sog. Datenbanktrigger, welche Veränderungen am Ausgangsdatenbestand der Datenbank kontinuierlich überwachen und im Falle einer Änderung von Ausgangsdaten eine entsprechende Auswerteaufforderung erzeugen. Die Datenbanktrigger sind vorzugsweise im Datenbankmanagementsystem (DBMS) der Datenbank implementiert. Alternativ oder zusätzlich kann auch das Programmmodul, beispielsweise eine Applikation, welches Ausgangsdaten in der Datenbank abspeichert bzw. ändert, entsprechende Auswerteaufforderungen selbständig generieren, wobei mit dem Abspeichern der Ausgangsdaten in der Datenbank gleichzeitig eine entsprechende Auswerteaufforderung erzeugt wird. Durch das Erkennen einer Änderung von Ausgangsdaten und das nachfolgende Erzeugen einer darauf basierenden Auswerteaufforderung können die von der Änderung betroffenen Auswertedaten vorteilhafterweise automatisch und kontinuierlich aktualisiert werden.
Es ist außerdem vorteilhaft, wenn das vorgegebene Ereignis ein Erreichen eines vorgegebenen Zeitpunkts oder einer vorgegebenen Zeitdauer ist. Dadurch können die Auswertedaten noch besser und effizienter aktuell gehalten werden. Das Erzeugen der Auswerteaufforderung erfolgt in diesem Fall zeitraumgesteuert. Die Auswertedaten können somit aufgrund der weiter schreitenden Zeit angepasst werden. Dazu ist es nicht notwendigerweise erforderlich, dass sich Ausgangsdaten ändern, neu hinzukommen oder gelöscht werden.
Vorzugsweise ist die Datenbank zusätzlich zum Abspeichern von Auswerteaufforderungen ausgestaltet. Die Steuerung ist dabei so ausgestaltet, dass sie jeweils eine der in der Datenbank abgespeicherten Auswerteaufforderungen auswählt und abhängig von der ausgewählten Auswerteaufforderung das in dem Speicher abgespeicherte Auswerteprogramm startet, welches dann in Abhängigkeit von der ausgewählten Auswerteaufforderung auf in der Datenbank abgespeicherte Ausgangsdaten zugreift. Dadurch kann das Erzeugen der Auswertedaten noch weiter verbessert und noch effizienter gestaltet werden.
Es ist außerdem bevorzugt, dass die Auswerteaufforderungen in einer Reihenfolge in der Datenbank gespeichert werden und die Steuerung hierbei so ausgestaltet ist, dass sie jeweils eine der in der Datenbank abgespeicherten Auswerteaufforderungen entsprechend dieser Reihenfolge auswählt. Dadurch kann das Erzeugen der Auswertedaten flexibel gestaltet und an verschiedene Anwendungen angepasst werden. Durch die Reihenfolge kann beispielsweise eine Priorität der Auswerteaufforderungen vorgegeben werden. Vorzugsweise werden die Auswerteaufforderungen nach dem Prinzip "first in, first out" (FIFO) abgespeichert und ausgewählt.
In einer weiteren vorteilhaften Ausgestaltung der Erfindung ist die Steuerung so ausgestaltet, dass nach einer Benutzeranfrage durch einen Benutzer die erzeugten Auswertedaten ausgegeben werden, wobei das Erzeugen der Auswertedaten zeitlich vor der Benutzeranfrage liegt. Der Benutzer erhält auf dieser Weise stets aktuelle Auswertedaten, die er nach Bedarf aus- und/oder verwerten kann. Beispielsweise kann er sich die aktuellen Auswertedaten auf einem Bildschirm und/oder einen Ausdruck grafisch visualisieren lassen.

Vorzugsweise ist die Steuerung so ausgestaltet, dass die erzeugten Auswertedaten in der Datenbank abgespeichert werden. Dadurch kann insbesondere eine gute Zugänglichkeit der Auswertedaten gewährleistet werden.

In einer weiteren vorteilhaften Ausgestaltung weist das System eine Auswertevorrichtung als eigenständige physikalische Einheit auf, welche den Speicher und die Steuerung umfasst. Die Datenbank und die Auswertevorrichtung sind hierbei zum Austauschen von Daten miteinander verbunden. Dadurch können die verschiedenen Funktionen, d.h. insbesondere das Auswerten und das Abspeichern der Ausgangsdaten, in den verschiedenen physikalischen Einheiten optimiert ausgestaltet werden.
In einer besonders bevorzugten Ausgestaltung der Erfindung ist ein Rechnernetzwerk vorhanden, das mehrere Rechnereinrichtungen enthält, die untereinander und mit der Datenbank zum Austauschen von Daten verbunden sind. Auf den Rechnereinrichtungen können verschiedene Applikationen eingerichtet sein, die Ausgangsdaten in der Datenbank abspeichern. Das Einbinden der Datenbank in das Rechnernetzwerk bietet eine besonders große Fülle an Einsatzmöglichkeiten. Im Falle einer Auswertevorrichtung als eigenständiger physikalischer Einheit kann diese vorteilhafterweise ebenfalls an das Rechnernetzwerk angebunden sein.
Vorzugsweise ist das erfindungsgemäße System als ein Krankenhausinformationssystem (HIS, CIS) ausgestaltet, wobei die Ausgangsdaten eine Vielzahl von Stamm- und/oder Bewegungsdaten, welche in einem Krankenhaus bei medizinischen und/oder logistischen und/oder administrativen Prozessen erzeugt werden, umfassen und die aus den Ausgangsdaten erzeugten Auswertedaten Kennzahlen dieser Prozesse im Krankenhaus umfassen. Die in einem Krankenhaus ablaufenden Prozesse sind besonders komplex, so dass der Einsatz der vorliegenden Erfindung besonders vorteilhaft ist, um trotz ständiger Änderungen in einer Vielzahl von Ausgangsdaten stets aktualisierte Auswertedaten zur Verfügung zu haben.
Ein weiterer besonderer Vorteil der Erfindung liegt darin, dass das System bzw. Verfahren Events, d.h. Auswerteaufforderungen, nutzen kann, die primär zum Zwecke der Kommunikation mit anderen Systemen, wie z.B. SAP, benötigt und erzeugt werden. Bei dem erfindungsgemäßen System bzw. Verfahren muss dadurch lediglich eine entsprechende Verwendung dieser Events konfiguriert werden.

Hierzu wird die Steuerung in der Weise angepasst, dass abhängig vom Eventtyp ein entsprechender Programmteil, der ggf. noch erstellt werden muss, aufgerufen wird, welcher unter Zugriff auf Ausgangsdaten Auswertedaten aktualisiert. So ist z.B. die Aufnahme eines Patienten in einem Krankenhausinformationssystem ein typisches Ereignis, bei welchem bereits zu Kommunikationszwecken ein Event erzeugt wird. Dieses bereits erzeugte Event kann nun gleichzeitig zur Erzeugung von Auswertedaten, welche vorzugsweise Kennzahlen dieser Prozesse im Krankenhaus umfassen, benutzt werden.
Weitere Vorteile der Erfindung ergeben sich aus der folgenden Beschreibung von Ausführungsbeispielen, wobei Bezug auf die beigefügten Zeichnungen genommen wird. Es zeigen:
- Fig. 1: eine schematische Darstellung eines ersten Ausführungsbeispiels eines erfindungsgemäßen Systems mit einem Rechnernetzwerk, bei dem verschiedene Client-Rechner direkt auf einen Datenbank- und eine Fileserver zugreifen können,
- Fig. 2: eine schematische Darstellung eines zweiten Ausführungsbeispiels eines erfindungsgemäßen Systems mit einem Rechnernetzwerk, bei dem verschiedene Client-Rechner auf Terminalserver zugreifen können, die wiederum mit einem Datenbank- und einem Fileserver verbunden sind,
- Fig. 3: eine schematische Darstellung einer mit dem Datenbankserver verbundenen Auswerteeinrichtung, wobei der Datenbankserver und die Auswerteeinrichtung Bestandteile der erfindungsgemäßen Systeme gemäß dem ersten und dem zweiten Ausführungsbeispiel sind,
- Fig. 4: ein Ablaufdiagramm eines Ausführungsbeispiels des erfindungsgemäßen Verfahrens,
- Fig. 5A,: B ein Vergleichsbeispiel zwischen einem Verfahren gemäß einem Stand der Technik und einem erfindungsgemäßen Verfahren, bei denen jeweils eine Anzahl von Übernachtungen von Patienten in einem Krankenhaus innerhalb eines Jahres bestimmt wird, und
- Fig. 6A -: F ein Anwendungsbeispiel des erfindungsgemäßen Verfahrens, bei dem Auswertedaten bereitgestellt werden, die in einem erfindungsgemäßen Krankenhausverwaltungssystem Informationen über mögliche abrechenbare Zusatzentgelte enthalten.

Im Folgenden werden, sofern nicht anders angegeben, für gleiche oder gleichwirkende Elemente gleiche Bezugszeichen verwendet.

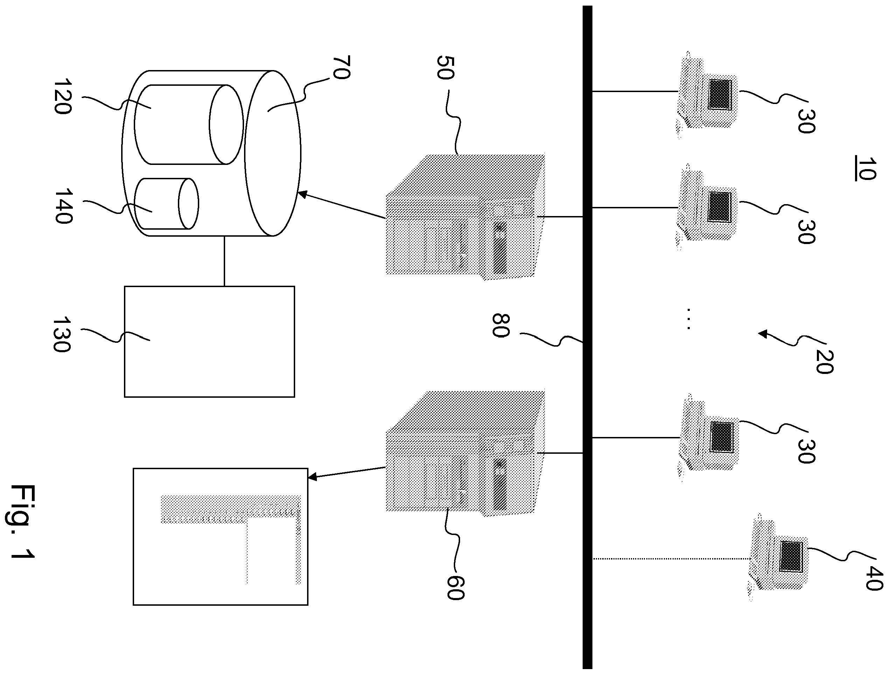

Fig. 1 zeigt eine schematische Darstellung eines ersten Ausführungsbeispiels des erfindungsgemäßen Systems 10. Das System 10 ist in diesem Beispiel ein Krankenhausverwaltungssystem, das zur Verwaltung und Durchführung einer Vielzahl von Aufgaben in einem Krankenhaus installiert ist.
Das System 10 umfasst ein Rechnernetzwerk 20 mit einer Vielzahl von verschiedenen, im Krankenhaus untergebrachten Rechnern 30 und einem beispielhaft über ein sog. Virtual Private Network (VPN) von außerhalb des Krankenhauses in das Netzwerk 20 integrierten Rechner 40. Die Rechner 30 und 40 sind Clients einer Client-Server-Architektur.
Das System 10 enthält ferner einen zentralen Datenbankserver 50 und einen zentralen Fileserver 60. Der Datenbankserver 50 enthält eine Datenbank 70, die hier beispielsweise eine Datenbank der Firma *Oracle* sein kann. Verschiedene, in dem Krankenhaus für die Verwaltung eingesetzte Applikationen sind auf dem Fileserver 60 installiert. Alternativ können die Applikationen auch direkt auf den Rechnern 30 und 40 installiert sein.
Die Rechner 30, 40, der Datenbankserver 50 und der Fileserver 60 sind jeweils an ein TCP/IP basiertes Netzwerk 80 angeschlossen, das hier ein sogenanntes Local Area Network (LAN) ist. Die Rechner 30, 40 können somit direkt auf den Datenbankserver 50 und den Fileserver 60 zugreifen.

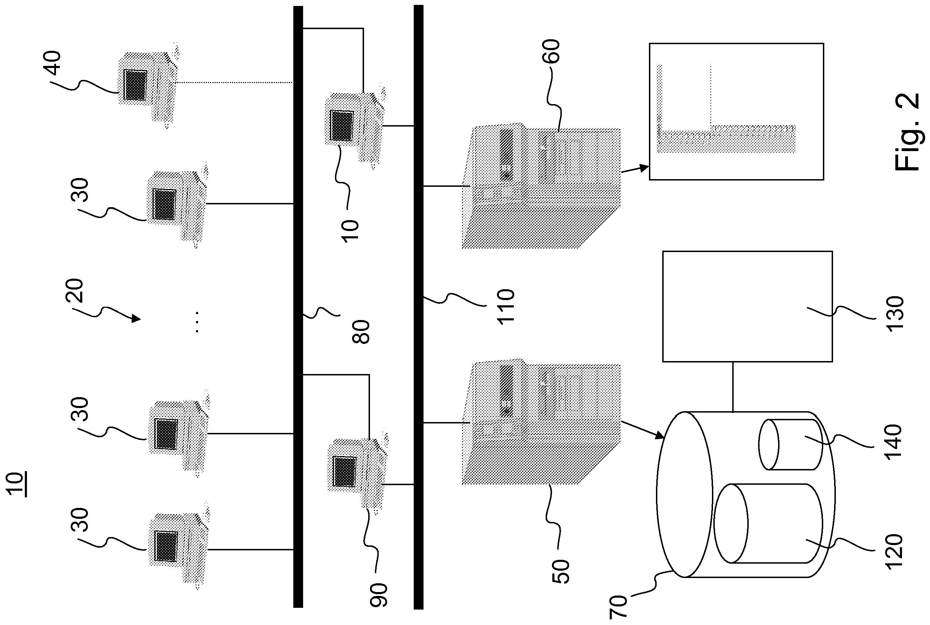

Fig. 2 zeigt eine schematische Darstellung eines zweiten Ausführungsbeispiels des erfindungsgemäßen Systems 10. Das System 10 des zweiten Ausführungsbeispiels entspricht weitgehend dem System 10 des ersten Ausführungsbeispiels, das anhand der Fig. 1 beschrieben wurde.
Im Unterschied zum System 10 gemäß dem ersten Ausführungsbeispiel sind die Rechner 30, 40 hier nicht direkt mit dem Datenbankserver 50 und dem Fileserver 60 verbunden. Vielmehr enthält das System 10 mehrere Terminalserver, von denen in der Fig. 2 stellvertretend zwei Terminalserver 90 und 100 dargestellt sind. Über die Terminalserver 90, 100 ist das LAN 80, an welches die Rechner 30, 40 angeschlossen sind, mit einem weiteren LAN 110 als TCP/IP basiertem Netzwerk verbunden. An das LAN 110 sind wiederum der Datenbankserver 50 und der Fileserver 60 angeschlossen. Die Rechner 30, 40 als Clients greifen somit in diesem Ausführungsbeispiel nicht direkt auf den Datenbankserver 50 und den Fileserver 60 zu, sondern über die Terminalserver 90, 100.

Im Folgenden werden das System 10 und seine Funktionsweise, sofern nicht anders vermerkt, sowohl für das erste als auch für das zweite Ausführungsbeispiel beschrieben.
Die Applikationen können beispielsweise beim Start einer Hauptapplikation beim System 10 gemäß dem ersten Ausführungsbeispiel auf die verschiedenen Rechner 30, 40 und beim System 10 gemäß dem zweiten Ausführungsbeispiel auf die Terminalserver 90, 100 kopiert oder installiert werden.
Für das System 10 gilt, dass die Clients 30, 40 über den Einsatz der auf dem Fileserver 60 installierten Applikationen Ausgangsdaten erzeugen können, die sie zentral in einem Datenbankbereich 120 der Datenbank 70 abspeichern. Bei den erzeugten Ausgangsdaten handelt es sich um Stamm- und Bewegungsdaten, die insbesondere von Benutzern des Systems 10 eingegeben werden.
Aus den in dem Datenbankbereich 120 abgespeicherten Ausgangsdaten sollen Auswertungen erstellt werden. Je nach Art und Informationsgehalt dieser Auswertungen müssen die Ausgangsdaten unter Umständen in komplizierten Berechnungsschritten zusammengefasst und interpretiert werden. Durch die Auswertungen werden aus den Ausgangsdaten Auswertedaten erzeugt.
Im vorliegenden Beispiel eines Einsatzes des Systems in einem Krankenhaus handelt es sich bei den Auswertedaten vorzugsweise um sog. Statistikdaten, welche dem Benutzer in Form von bestimmten Kennzahlen Aufschluss über verschiedene Vorgänge im Krankenhaus, wie z.B. zur medizinischen Pflege, zum Rechnungswesen oder zur Logistik, geben.

Zum Auswerten der abgespeicherten Ausgangsdaten und zum Erzeugen der Auswertedaten weist das System 10 eine Auswerteeinrichtung 130 auf, die eine eigenständige physikalische Einheit darstellt. Die Auswerteeinrichtung 130 ist mit dem Datenbankserver 50 zum Austauschen von Daten verbunden. Die von der Auswerteeinrichtung 130 erzeugten Auswertedaten können vorteilhafterweise ein eigenes Datenmodell aufweisen, das von demjenigen der Ausgangsdaten getrennt ist. Die Auswertedaten werden in einem eigenen Datenbankbereich 140 in der Datenbank 70 abgespeichert.
Die in dem Bereich 140 abgespeicherten Auswertedaten können von einem Benutzer abgerufen werden, beispielsweise um sie weiter zu verarbeiten und/oder auszuwerten, insbesondere um sie visualisieren zu lassen. Der Benutzer kann dazu über einen der Rechner 30, 40 auf den Datenbankserver 50 und die in seinem Bereich 140 abgespeicherten Auswertedaten zugreifen. Die Visualisierungen der Auswertedaten können beispielsweise auf einem Monitor der Rechner 30, 40 dargestellt oder auf Papier ausgedruckt werden. Es ist auch möglich, die Auswertedaten in Dateiform, beispielsweise in einem CSV- oder XML-Format, auszugeben.

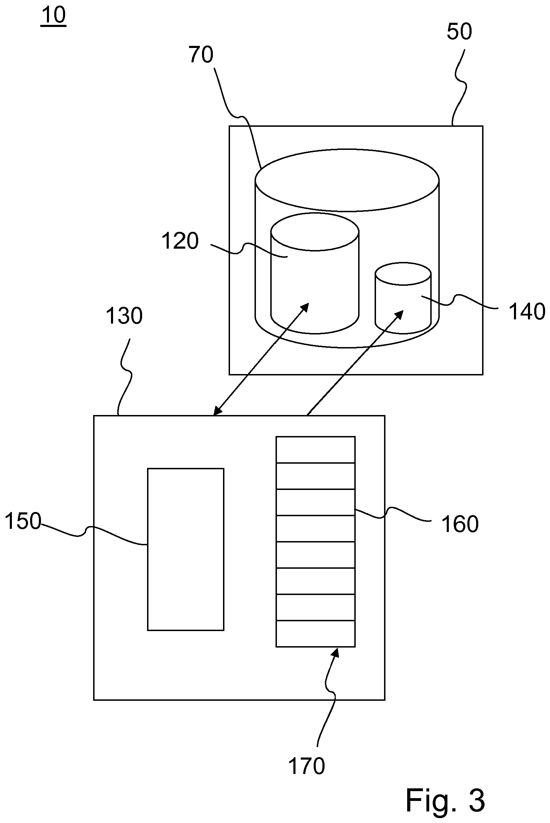

Fig. 3 zeigt eine etwas detailliertere schematische Darstellung der Auswerteeinrichtung 130, die mit dem Datenbankserver 50 verbunden ist. Die Auswerteeinrichtung 130 enthält eine Steuerung 150 zum Steuern der Auswerteeinrichtung 130 und insbesondere zum Steuern des Auswertens der in dem Datenbankbereich 120 abgespeicherten Ausgangsdaten und des Erzeugens der Auswertedaten. Die Auswerteeinrichtung 130 enthält ferner einen Speicher 160, in dem ein Auswerteprogramm abgespeichert ist, mit dem die Ausgangsdaten ausgewertet werden.
Das Auswerteprogramm ist in mehrere Programmteile 170 unterteilt, die auch als Plugins bezeichnet werden können. Die Programmteile 170 können prinzipiell in verschiedenen Programmiersprachen bereitgestellt werden. Im vorliegenden Fall sind die Programmteile 170 in der Programmiersprache *Java* programmiert.

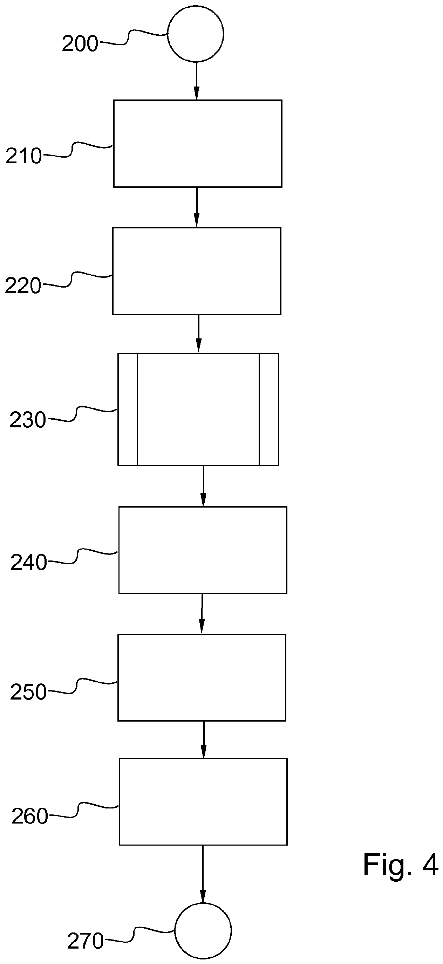

Fig. 4 zeigt ein Ablaufdiagramm eines Ausführungsbeispiels eines erfindungsgemäßen Verfahrens. Das erfindungsgemäße Verfahren beginnt bei einem Verfahrensschritt 200.
Ein Eintreten eines vorgegebenen Ereignisses wird durch einen Schritt 210 repräsentiert. Ein solches vorgegebenes Ereignis kann eine Änderung von Ausgangsdaten in der Datenbank 70 oder ein Erreichen eines vorgegebenen Zeitpunkts oder einer vorgegebenen Zeitdauer sein. In dem Bereich 120 der Datenbank 70 können sich hierbei sowohl Stamm- als auch Bewegungsdaten ändern.
Eine solche Änderung von Stammdaten kann in der Krankenhausumgebung der hier beschriebenen Ausführungsbeispiele beispielsweise dann vorliegen, wenn für eine Fachabteilung des Krankenhauses, z.B. die Intensivstation, ein Kennzeichen "Entlasstag aus dem Krankenhaus" von dem Status "nicht zählen" auf den Status "mitzählen" geändert wird. Dies bedeutet, dass nun als Auswertedaten für alle Patientenfälle, die von dieser Fachabteilung entlassen wurden, der jeweilige Entlasstag nachträglich zu bestimmen und entsprechend zu ändern ist.

Eine Änderung von Bewegungsdaten liegt z.B. dann vor, wenn für einen Patient nachträglich der Aufenthalt in einer Fachabteilung verlängert oder verkürzt wird. In diesem Fall sind die Auswertedaten für den Aufenthalt dieses Patientenfalles neu zu ermitteln. Falls der Aufenthalt verkürzt wurde, müssen zuviel berücksichtigte Tage gelöscht werden. Falls der Aufenthalt verlängert wurde, müssen entsprechend zusätzliche Tage ermittelt und berücksichtigt werden.
Ein weiteres Beispiel für eine Änderung von Ausgangsdaten ist der Neueintrag eines Aufenthalts eines Patienten. In diesem Fall sind die Auswertedaten insofern zu ändern, als erstmals Ausgangsdaten über die Aufnahme und den Fachabteilungsaufenthalt, z.B. eine Diagnose, angelegt werden. Eine Änderung der Ausgangsdaten erfolgt beispielsweise auch dann, wenn ein Patientenaufenthalt storniert wird. Unter Umständen müssen hier alle Einträge für diesen Patientenfall aus dem Datenbankbereich 140 gelöscht oder als ungültig markiert werden.
Des Weiteren treten auch bei einer Beendigung eines Patientenaufenthalts in dem Krankenhaus Änderungen der Ausgangsdaten auf. In dem Datenbankbereich 140 müssen somit Informationen zur Entlassung des Patienten vermerkt werden. Eventuell zuviel berücksichtigte Tage müssen entsprechend gelöscht oder als ungültig markiert werden.
Ein Beispiel für ein Erreichen eines vorgegebenen Zeitpunkts oder einer vorgegebenen Zeitdauer liegt dann vor, wenn nach einem Erzeugungszeitpunkt von Ausgangsdaten ein neuer Tag eingetreten ist. Es sind dann Auswertedaten für den Aufenthalt aller Patientenfälle, die zu dem Tageswechsel weiterhin im Krankenhaus sind, neu zu ermitteln. Die Aufenthaltsdauer der verschiedenen Patientenfälle im Krankenhaus ist um einen Tag zu erhöhen. Im letzten Fall muss nicht notwendigerweise auch eine Änderung von Ausgangsdaten aufgetreten sein.
Es ist eine Vielzahl von weiteren vorgegebenen Ereignissen möglich, die zu einem Erzeugen von Auswertedaten führen können.
Auf der Datenbank 70 sind sog. Datenbanktrigger eingerichtet, durch welche Veränderungen am Ausgangsdatenbestand der Datenbank 70 kontinuierlich überwacht und erfasst werden und anschließend in einem Schritt 220 eine entsprechende Auswerteaufforderung zum Auswerten von Ausgangsdaten erzeugt wird.

Eine solche Auswerteaufforderung kann auch als "Event" bezeichnet werden. Die Auswerteaufforderung wird in diesem Beispiel in einer speziellen Tabelle für Auswerteaufforderungen abgespeichert.
Alternativ oder zusätzlich kann in Schritt 220 auch mittels eines Programmmoduls, beispielsweise einer der auf dem Fileserver 60 installierten Applikation, welches bestimmte vordefinierte Operationen ausführt, ein Event, d.h. eine Auswerteaufforderung, erzeugt und in der dafür vorgesehenen speziellen Tabelle abgespeichert werden. Ein Beispiel dafür ist die Neuaufnahme eines Patienten im Krankenhaus. Dabei müssen u.a. der Name, die Anschrift und eine Diagnose erfasst werden. Es können somit von dieser Neuerfassung des Patienten mehrere Tabellen der Datenbank 70 mit Ausgangsdaten betroffen sein.
Die spezielle Tabelle für Auswerteaufforderungen ist vorzugsweise in einer in der Datenbank 70 befindlichen Tabelle abgespeichert.
Vorzugsweise wird von der Datenbank bzw. dem Programmmodul abhängig von der Art des jeweils eingetretenen Ereignisses ein spezieller Typ der Auswerteaufforderung erzeugt. Der Typ der Auswerteaufforderung ist somit einem oder mehreren bestimmten vorgegebenen Ereignissen zugeordnet.
Mehrere Auswerteaufforderungen können in der Tabelle in einer bestimmten Reihenfolge abgespeichert werden. Diese Reihenfolge bestimmt sich beispielsweise nach dem Typ, dem die jeweiligen Auswerteaufforderungen angehören, einer vorgegebenen Priorität und/oder nach dem Prinzip "first in, first out" (FIFO).
In einem weiteren Schritt 230 des Verfahrens nach Fig. 4 werden die in der speziellen Tabelle abgespeicherten Auswerteaufforderungen entsprechend der ggf. vorgegebenen Reihenfolge von der Steuerung 150 aus der Tabelle ausgelesen.

In einem nachfolgenden Schritt 240 greift die Steuerung 150 in Abhängigkeit von der Auswerteaufforderung, insbesondere in Abhängigkeit von dem Typ der Auswerteaufforderung, auf einen der Programmteile 170 zu, welcher in einem nachfolgenden Schritt 250 ausgeführt wird, in welchem die Auswertedaten erzeugt werden. Die in dem Speicher 160 abgespeicherten Programmteile 170 sind somit den Auswerteaufforderungen, insbesondere den Typen von Auswerteaufforderungen, zugeordnet. Durch den jeweiligen, von der Steuereinrichtung 150 ausgewählten und ausgeführten Programmteil 170 sind die Auswertedaten festgelegt, die mittels des jeweiligen Programmteils 170 erzeugt werden.
In einem nachfolgenden Schritt 260 werden die erzeugten Auswertedaten dann in den Datenbankbereich 140 übertragen und dort abgespeichert. Gegebenenfalls in dem Bereich 140 bereits vorhandene "veraltete" Auswertedaten werden gelöscht oder durch die neuen Auswertedaten überschrieben. In einem Schritt 270 wird das erfindungsgemäße Verfahren beendet.
Bei dem vorliegenden erfindungsgemäßen System bzw. Verfahren werden die Auswertedaten somit "Event-basiert" aktualisiert, d.h. die Auswertedaten werden abhängig vom Eintreten eines bestimmten Ereignisses und der damit verbundenen Generierung eines bestimmten Events, d.h. einer Auswerteaufforderung, aktualisiert.
Es ist erfindungsgemäß prinzipiell möglich, auf die Zuteilung eines Typs der Auswerteaufforderung zu verzichten. In diesem Fall erfolgt eine Zuordnung der Auswerteaufforderung einerseits zu dem erfassten vorgegebenen Ereignis und andererseits zu einem oder mehreren der Programmteile 170.
Erfindungsgemäß werden die Auswertedaten regelmäßig dann erzeugt, wenn ein vorgegebenen Ereignis erfasst wird. Das Erzeugen der Auswertedaten erfolgt über die Erzeugung der Auswerteaufforderung und die geeignete Zuordnung und Auswahl der Programmteile 170. Es kann somit vorteilhafterweise sichergestellt werden, dass stets aktuelle Auswertedaten in dem Bereich 140 der Datenbank 70 abgespeichert sind. Ein Benutzer, der Auswertedaten aus dem Bereich 140 abruft, erhält daher stets aktuelle Auswertedaten. Es ist nicht erforderlich, bei Abrufen von Auswertedaten durch den Benutzer, diese erst aktuell neu zu erzeugen. Die Belastungen der Steuerung 150 durch das Erzeugen der Auswertedaten können daher weitgehend über einen längeren Zeitraum gestreckt werden. Eine punktuell starke Belastung der Steuerung 150, wie sie beim Neuerzeugen der Auswertedaten bei einer Anfrage des Benutzers notwendig wäre, wird somit vermieden.
Es wird erfindungsgemäß ferner gewährleistet, dass nur diejenigen Auswertedaten erzeugt werden, für die es aufgrund des jeweils erfassten vorgegebenen Ereignisses erforderlich ist, um sie aktuell zu halten.
Durch die erfindungsgemäße Vorerzeugung der Auswertedaten steht einem Benutzer die Steuereinrichtung 150 oder einer der Rechner 30, 40, auf dem eine aktuelle Auswertung der Ausgangsdaten benötigt wird, sehr schnell wieder für andere Zwecke zur Verfügung.

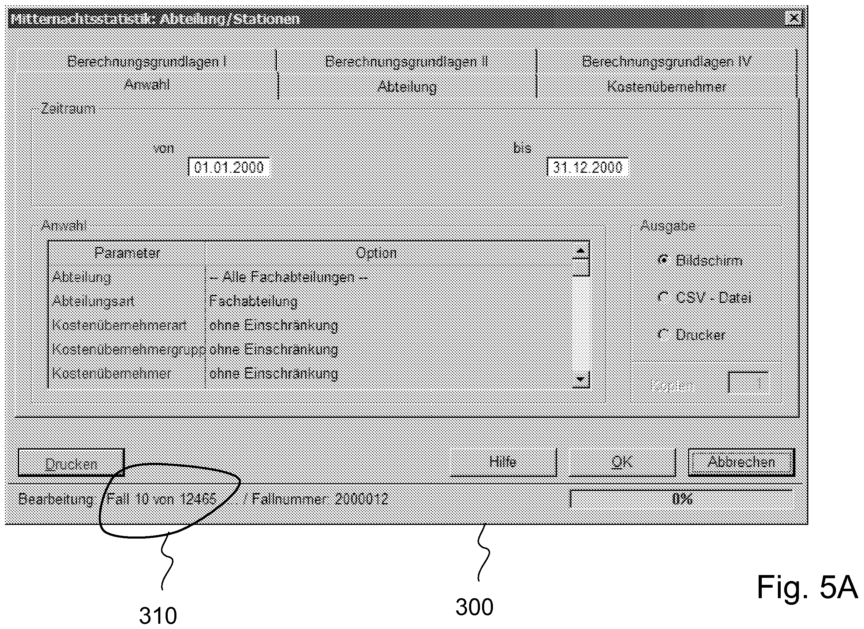

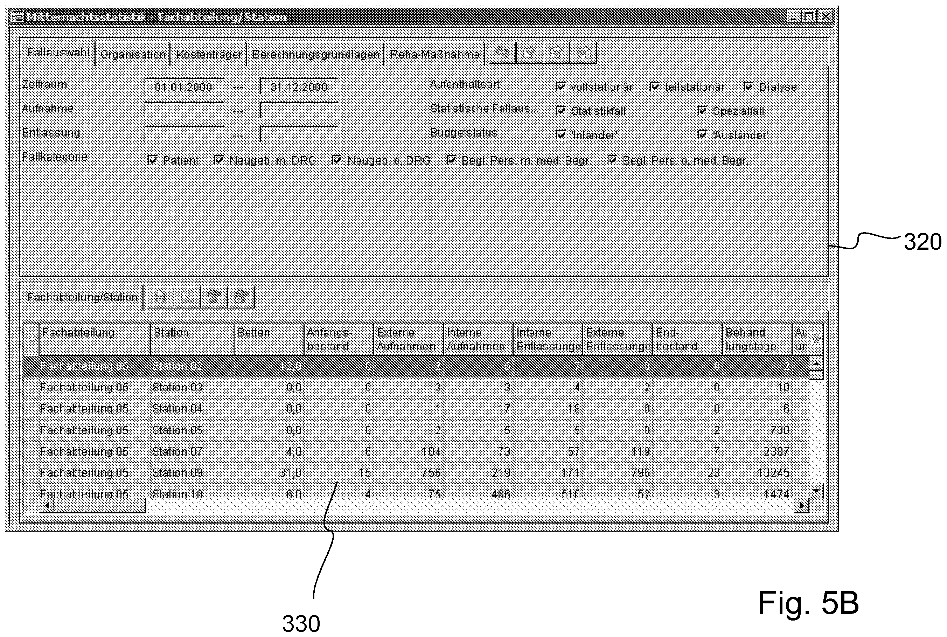

Die Fig. 5A und 5B zeigen dazu ein Vergleichsbeispiel zwischen einem Verfahren gemäß einem Stand der Technik (Fig. 5A) und einem erfindungsgemäßen Verfahren (Fig. 5B). Bei dem Vergleichsbeispiel wird nach beiden Verfahren jeweils eine Anzahl von Übernachtungen von Patienten in einem Krankenhaus innerhalb eines Jahres, hier das Jahr 2000, bestimmt.
Fig. 5A zeigt eine Maske 300, die dem Benutzer auf seinem Monitor angezeigt wird. In dieser Vorgehensweise nach dem Stand der Technik werden nach der Anfrage des Benutzers alle Patientenfälle und die geforderten Auswertedaten nacheinander neu berechnet. Dies sind hier insbesondere eine Dauer des Aufenthalts auf verschiedenen Fachabteilungs- und Stationskombinationen, Verlegungsinformationen, Informationen, aus welchem Krankenhaus der jeweilige Patient ggf. gekommen ist oder wohin er verlegt wurde, Informationen über Beurlaubungen des jeweiligen Patienten usw.
Der jeweils aktuell erfasste Patient, der als "Fall" bezeichnet wird, wird in der Maske in einem Feld 310 angezeigt, in dem ein Zähler die Patientenfälle einzeln hochzählt. Im Beispiel nach Fig. 5A ist dies momentan der Patientenfall 10 von 12465 Patientenfällen insgesamt. Im vorliegenden Beispiel nach Fig. 5A benötigt das System 10 ca. 5 Minuten und 30 Sekunden, bis das komplette Resultat erzeugt wurde und die gewünschten Auswertedaten dem Benutzer zur Verfügung stehen.
Fig. 5B zeigt eine Maske 320, in der die gewünschten Auswertedaten bereits nach ca. 30 Sekunden in einem Feld 330 angezeigt werden. Dies ist daher möglich, da die aktuellen Auswertedaten bereits in dem Datenbankbereich 140 abgespeichert sind. Die gewünschten Auswertedaten müssen somit nur aus der Datenbank 70 ausgewählt und zum Rechner des Benutzer übertragen werden. Es ist keine komplexe Berechnung der gewünschten Auswertedaten mehr erforderlich.

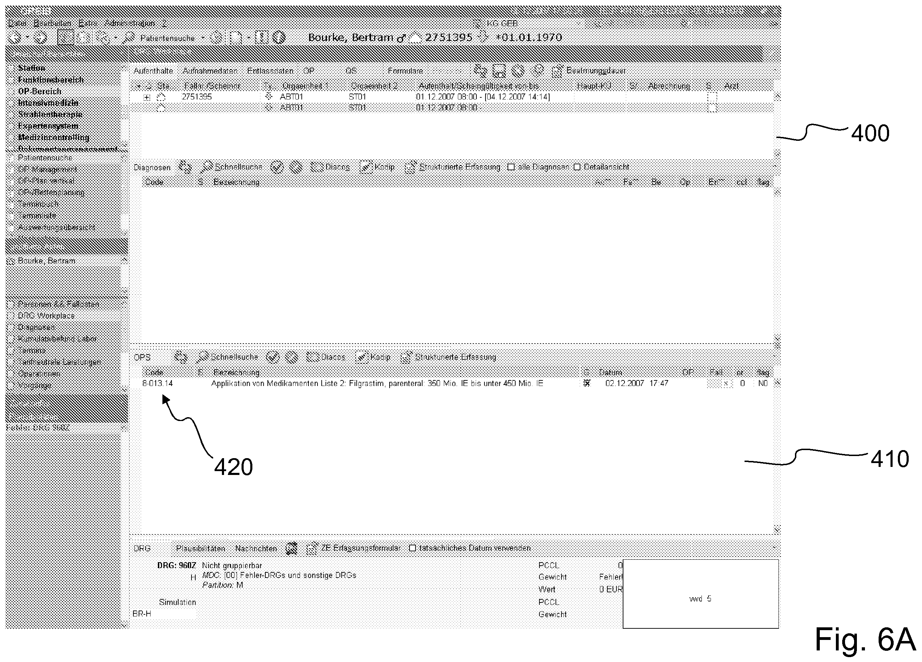

Fig. 6A-F zeigen ein Anwendungsbeispiel der vorliegenden Erfindung. Bei diesem Anwendungsbeispiel werden Auswertedaten bereitgestellt, die in dem erfindungsgemäßen Krankenhausverwaltungssystem 10 Informationen über mögliche abrechenbare Zusatzentgelte enthalten. Zusatzentgelte sind Leistungen, die neben anderen "normalen" Leistungen bei einem Patientenfall vom Krankenhaus in Rechnung gestellt werden können. In Deutschland können Zusatzentgelte beispielsweise parallel zu einer Fallpauschale abgerechnet werden.
Im vorliegenden Beispiel möchte der Benutzer zum Zeitpunkt des Aufenthaltes einer Vielzahl von Patientenfällen im Krankenhaus wissen, welche potentiellen Zusatzentgelte diese Patientenfälle einbringen können. Anstatt Auswertedaten mit diesen Informationen bei einer entsprechenden Anfrage erneut zu berechnen, wird erfindungsgemäß die oben beschriebene Vorgehensweise mittels Auswerteaufforderungen eingesetzt. Gespeichert werden die als Auswertedaten ermittelten potentiellen Zusatzentgelte in einer Tabelle mit dem Namen ZEPOT. Zusatzentgelte ermitteln sich prinzipiell aus unterschiedlichen Informationen. Dazu zählen z.B. hinterlegte Operationsschlüssel (OPS), Alter des Patientenfalls, Aufnahmedatum usw. OPS sind Prozedurencodes, die auf der deutschen Adaption des internationalen WHO-ICPM (World Health Organization-International Classification of Procedures in Medicine) Kataloges basieren. Beschrieben und dargestellt wird die aus der Erfassung eines OPS resultierende Programmabfolge.

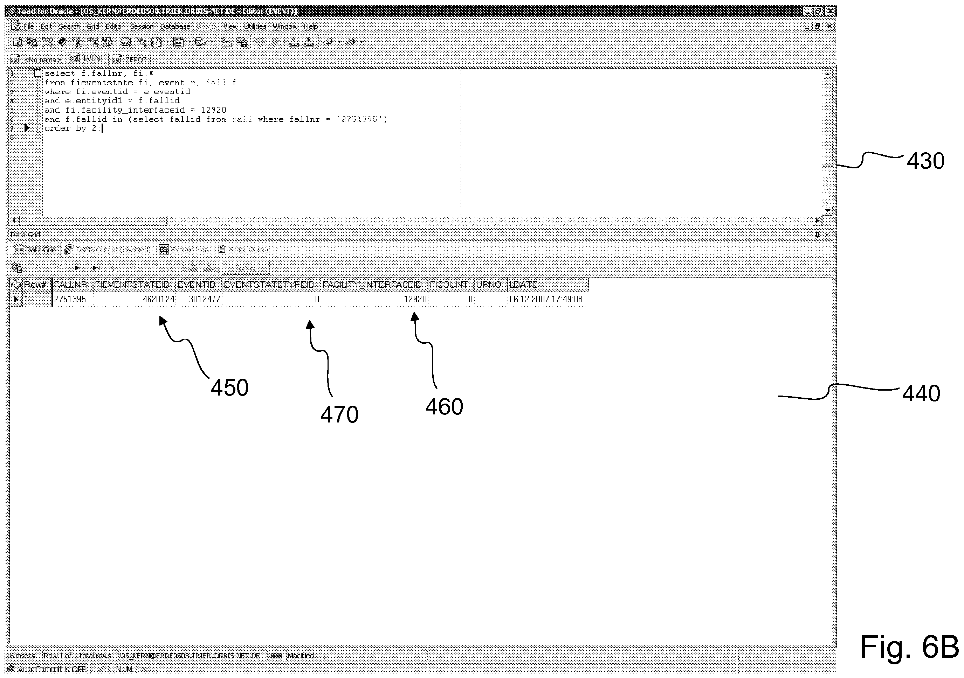

Ausgangspunkt für das Anwendungsbeispiel gemäß der Fig. 6A-F ist die Erfassung eines OPS im System 10 zum Patientenfall mit einer Fallnummer 2751395.
Fig. 6A zeigt eine Maske 400, die auf einem Monitor während des Ablaufs des erfindungsgemäßen Verfahrens angezeigt wird und vom Benutzer bedient werden kann. Zunächst wird ein OPS als geändertes Ausgangsdatum durch die Steuerung 150 erfasst. Dieser OPS wird dem Benutzer in einem Feld 410 angezeigt und ist hier mit dem Bezugszeichen 420 versehen. Zum Erfassen des OPS gibt es prinzipiell mehrere Möglichkeiten, wie z.B. über einen Mengenkalkulator, eine manuelle Eingabe oder über einen Transfer via externer Schnittstelle.
Durch das Erfassen und Codieren des OPS wird eine Auswerteaufforderung ausgelöst und erzeugt. Dies geschieht zunächst unabhängig davon, ob der OPS relevant ist für das Bestimmen des Zusatzentgelts oder nicht. Fig. 6B zeigt eine Maske 430 einer Tabelle FIEVENTSTATE, die eine spezielle Tabelle für das Abspeichern von Auswerteaufforderungen darstellt. Die Maske 430 weist ein Feld 440 mit einem Eintrag 450 auf. Der Eintrag 450 stellt eine Auswerteaufforderung mit einer FIEVENTSTATEID 4620124 dar. Die Tabelle FIEVENTSTATE gibt zusätzlich an, ob die Auswerteaufforderung für einen bestimmten Programmteil 170 des Auswerteprogramms in die Tabelle FIEVENTSTATE geschrieben wurde. Der Programmteil 170, der für die Erzeugung der Auswertedaten mit den Informationen über die potentiellen Zusatzentgelte zuständig ist, hat hier die ID 12920. Dies ist in der Tabelle FIEVENTSTATE in der Spalte FACILITY_INTERFACEID dargestellt und mit dem Bezugszeichen 460 versehen. In der Spalte EVENTSTATETYPEID des Feldes 440 findet man den Eintrag 0. Dieser ist hier mit dem Bezugszeichen 470 versehen und bedeutet, dass der Eintrag 450 von der Auswerteeinrichtung 130 noch nicht bearbeitet wurde. Der zugehörige Programmteil 170 wurde noch nicht aufgerufen.

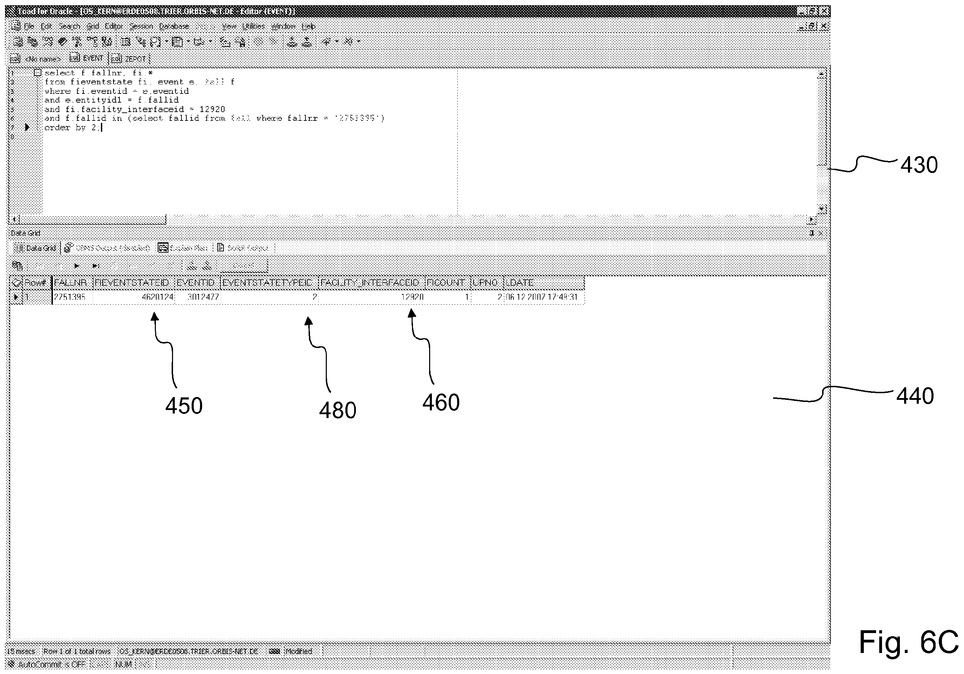

Fig. 6C zeigt erneut die Maske 430, wobei hier allerdings in der Spalte EVENTSTATETYPEID des Feldes 440 der Eintrag 2 steht. Dies ist mit dem Bezugszeichen 480 bezeichnet und bedeutet, dass die Auswerteaufforderung unter dem Eintrag 450 nunmehr bearbeitet wurde. Die Auswertedaten wurden mittels des zugewiesenen, mit der ID 12920 versehenen Programmteils 170 erzeugt.

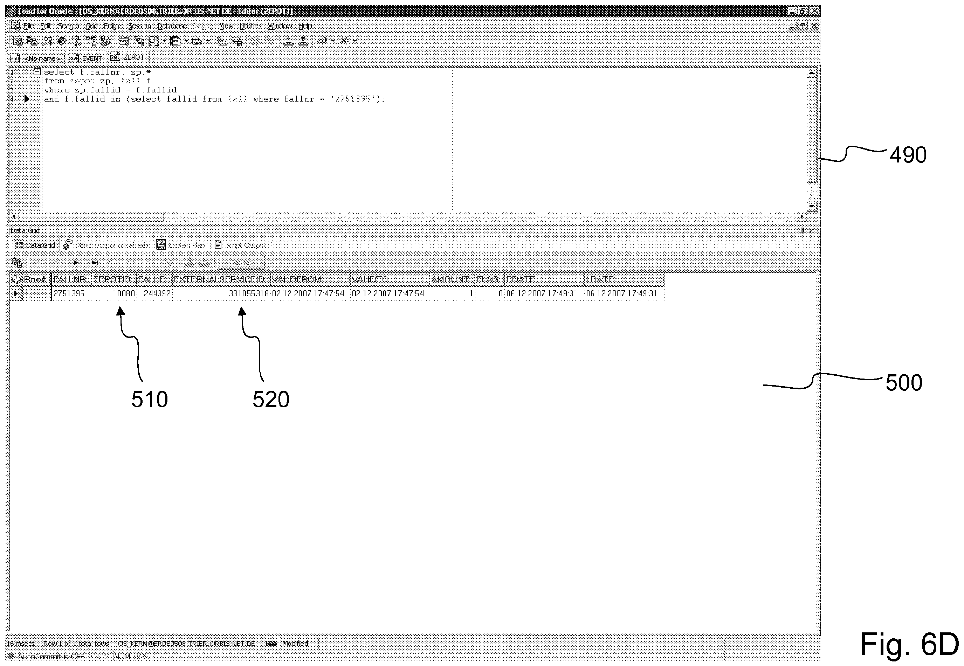

Fig. 6D zeigt eine Maske 490, in der in einem Feld 500 eine Tabelle ZEPOT dargestellt ist. Da der OPS hier so gewählt wurde, dass er zu einem Zusatzentgelt führt, enthält die Tabelle ZEPOT einen Eintrag 510 mit einem Zusatzentgelt zum betrachteten Patientenfall. Der Eintrag 510 stellt insofern ein Auswertedatum dar. Das Zusatzentgelt verbirgt sich hinter einem Eintrag ZEPOT.EXTERNALSERVICEID, der mit dem Bezugszeichen 520 versehen ist. Dieser Schlüssel 520 führt als Fremdschlüssel zum eigentlichen Text der abrechenbaren Leistung.

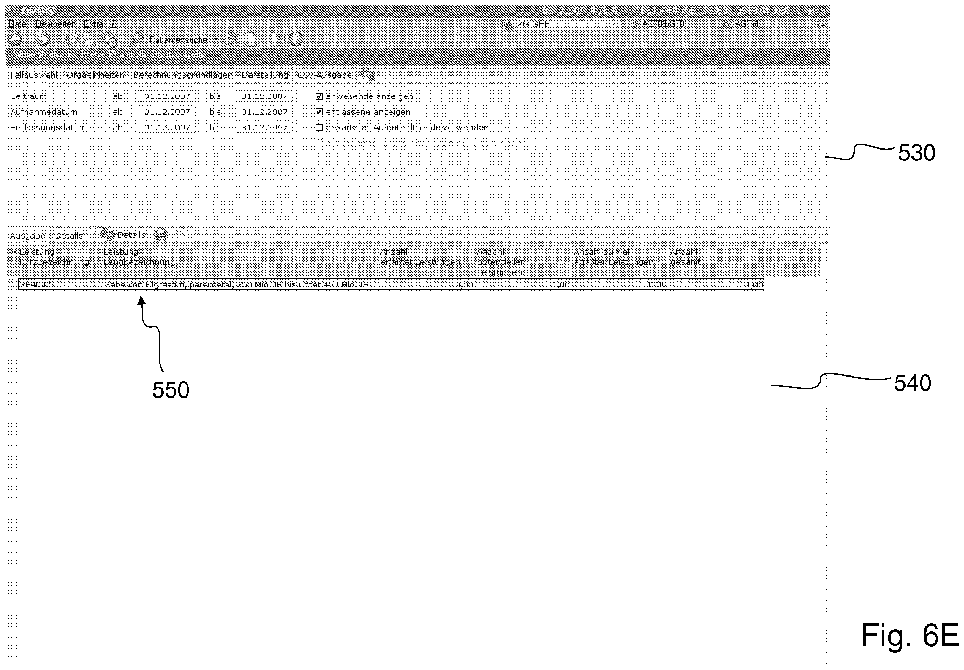

Fig. 6E zeigt eine Maske 530, die auf dem Monitor nach der Bearbeitung einer Anfrage zur Darstellung der potentiellen Zusatzentgelte durch das System 10 angezeigt wird. Die Maske 530 enthält in einem Feld 540 in Textform die Gesamtliste über das Zusatzentgelt, das abgerechnet werden kann. Im vorliegenden Beispiel ist das eine Angabe 550, mit der ein Zusatzentgelt durch eine Gabe von Filgrastim abgerechnet werden kann.

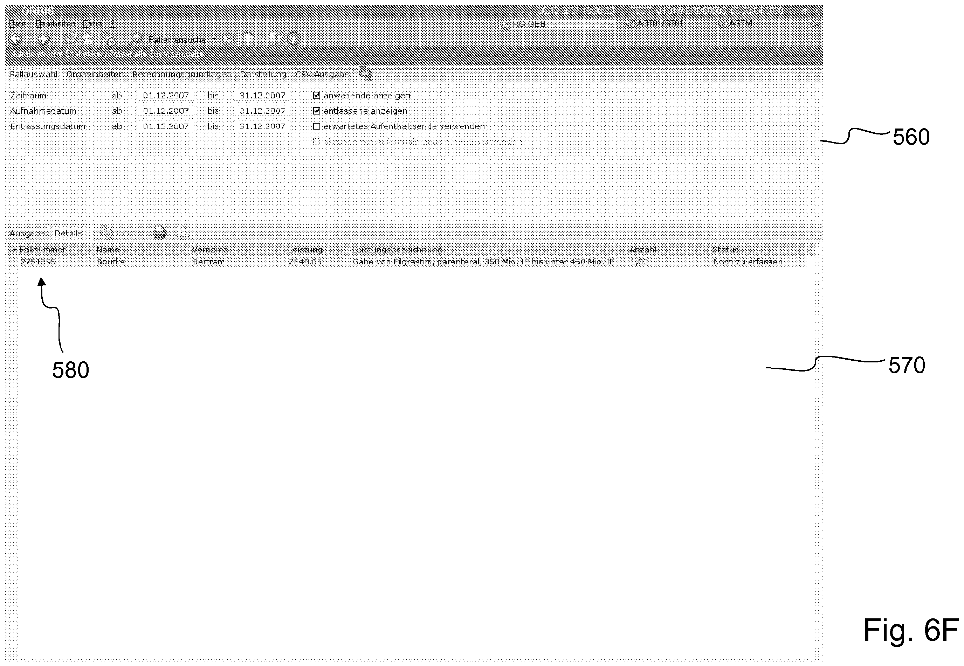

Fig. 6F zeigt eine Maske 560, die ebenfalls auf dem Monitor nach der Bearbeitung einer Anfrage zur Darstellung der potentiellen Zusatzentgelte durch das System 10 angezeigt wird. Die Maske 560 enthält in einem Feld 570 in Textform das bereits in dem Feld 540 angezeigte Gesamtergebnis, hier allerdings bezogen auf den Patientenfall mit der bereits oben genannten Fallnummer 2751395. Dies ist in der Fig. 6F durch ein Bezugszeichen 580 verdeutlicht.

## Patentansprüche

1. Verfahren zum Erzeugen von Auswertedaten, bei dem
- Ausgangsdaten, welche in einem Krankenhaus bei medizinischen und/oder logistischen und/oder administrativen Prozessen erzeugt werden, in einer Datenbank (70) abgespeichert werden,
- durch einen Datenbanktrigger Änderungen der in der Datenbank (70) gespeicherten Ausgangsdaten kontinuierlich überwacht werden und im Falle einer Änderung von Ausgangsdaten eine Auswerteaufforderung zum Auswerten von Ausgangsdaten erzeugt wird, durch ein von einem Programmmodul durchgeführtes Verfahren Ausgangsdaten in der Datenbank (70) abgespeichert oder geändert werden und dabei gleichzeitig eine Auswerteaufforderung zum Auswerten von Ausgangsdaten erzeugt wird und/oder bei Eintreten eines vorgegebenen Ereignisses in Form eines Erreichens eines vorgegebenen Zeitpunkts oder einer vorgegebenen Zeitdauer eine Auswerteaufforderung zum Auswerten von Ausgangsdaten erzeugt wird, wobei die Auswerteaufforderung zum Auswerten von Ausgangsdaten in einer in der Datenbank (70) befindlichen Tabelle abgespeichert wird,
- überprüft wird, ob eine Auswerteaufforderung erzeugt und in der Tabelle abgespeichert worden ist, und im Falle des Vorhandenseins einer Auswerteaufforderung ein in einem Speicher (160) abgespeichertes Auswerteprogramm zur Durchführung eines Auswerteverfahrens in Abhängigkeit von der Auswerteaufforderung gestartet wird und
- das Auswerteverfahren in Abhängigkeit von der Auswerteaufforderung auf in der Datenbank (70) abgespeicherte Ausgangsdaten zugreift und aus den Ausgangsdaten Auswertedaten, welche Kennzahlen der medizinischen und/oder logistischen und/oder administrativen Prozesse im Krankenhaus umfassen, erzeugt.

2. Verfahren nach Anspruch 1, wobei das Vorhandensein einer Auswerteaufforderung kontinuierlich überprüft wird, wodurch das in dem Speicher (160) abgespeicherte Auswerteprogramm zur Durchführung des Auswerteverfahrens unmittelbar nach dem Erzeugen der Auswerteaufforderung gestartet wird.

3. Verfahren nach Anspruch 1 oder 2, wobei das Auswerteverfahren mehrere unterschiedliche Verfahrensteile (170) zum Auswerten unterschiedlicher Ausgangsdaten aufweist, wobei
- in Abhängigkeit von dem vorgegebenen Ereignis unterschiedliche Typen von Auswerteaufforderungen erzeugt werden und
- in Abhängigkeit von dem jeweils erzeugten Typ der Auswerteaufforderung einer der Verfahrensteile (170) gestartet wird.

4. Verfahren nach Anspruch 3, wobei in Abhängigkeit von dem gestarteten Verfahrensteil (170) auf in der Datenbank (70) abgespeicherte Ausgangsdaten zugegriffen wird.

5. Verfahren nach Anspruch 3 oder 4, wobei bei einem zwei- oder mehrmaligen Eintreten von vorgegebenen Ereignissen im Zusammenhang mit einem bestimmten Auswertungsobjekt zwei bzw. mehrere Auswerteaufforderungen erzeugt werden und nur für eine dieser Auswerteaufforderungen einer der Verfahrensteile (170) gestartet wird, wenn die übrigen Auswerteaufforderungen ein Starten des selben Verfahrensteils (170) zur Folge hätten.

6. Verfahren nach einem der vorhergehenden Ansprüche, wobei jeweils eine der in der Datenbank (70) abgespeicherten Auswerteaufforderungen ausgewählt und abhängig von der ausgewählten Auswerteaufforderung das in dem Speicher (160) abgespeicherte Auswerteprogramm zur Durchführung des Auswerteverfahrens gestartet wird und das Auswerteverfahren in Abhängigkeit von der Auswerteaufforderung auf in der Datenbank (70) abgespeicherte Ausgangsdaten zugreift.

7. Verfahren nach Anspruch 6, wobei die Auswerteaufforderungen in einer Reihenfolge in der Datenbank (70) abgespeichert werden und jeweils eine der in der Datenbank (70) abgespeicherten Auswerteaufforderungen entsprechend dieser Reihenfolge ausgewählt wird.

8. Verfahren nach einem der vorhergehenden Ansprüche, wobei nach einer Benutzeranfrage durch einen Benutzer die erzeugten Auswertedaten ausgegeben werden, wobei das Erzeugen der Auswertedaten zeitlich vor der Benutzeranfrage liegt.

9. Verfahren nach einem der vorhergehenden Ansprüche, wobei die erzeugten Auswertedaten in der Datenbank (70) abgespeichert werden.

10. Verfahren nach einem der vorhergehenden Ansprüche, wobei mindestens eine Auswerteaufforderung zum Zwecke des Datenaustausches des Systems mit einem weiteren System dient.

11. System (10) zum Erzeugen von Auswertedaten, wobei das System (10) zur Durchführung des Verfahrens nach einem der vorangehenden Ansprüche ausgebildet ist.

12. System nach Anspruch 11, wobei ein Rechnernetzwerk (20) vorhanden ist, das mehrere Rechnereinrichtungen (30, 40) enthält, die untereinander und mit der Datenbank (70) zum Austauschen von Daten verbunden sind.

13. System nach Anspruch 11 oder 12, welches als ein Krankenhausinformationssystem (HIS, CIS) ausgestaltet ist.

14. Computerprogamm, welches, wenn es auf einem Datenverarbeitungssystem ausgeführt wird, jeden der Schritte des Verfahrens gemäß einem der Ansprüche 1 bis 10 durchführt.

## Claims

1. Method for producing evaluation data, wherein
- output data, which are produced in a hospital in medical and/or logistical and/or administrative processes, are stored in a database (70),
- changes of the output data stored in the database (70) are continuously monitored by a database trigger and in case of a change of output data an evaluation request for evaluating output data is generated, output data are stored or modified in the database (70) by a process executed by a program module, whereby an evaluation request for evaluating output data is generated simultaneously and/or an evaluation request for evaluating output data is generated when a predetermined event occurs in the form of a predetermined point of time or a predetermined duration being reached, wherein the evaluation request for evaluating output data is stored in a table located in the database (70),
- it is checked whether an evaluation request has been generated and has been stored in the table and in case an evaluation request is present, an evaluation program stored in a memory (160) for executing an evaluation method depending on the evaluation request is started and
- the evaluation method accesses output data stored in the database (70) depending on the evaluation request and generates evaluation data from the output data, said evaluation data comprising key performance indicators of the medical and/or logistical and/or administrative processes in the hospital.

2. Method according to claim 1, wherein the presence of an evaluation request is checked continuously so that the evaluation program stored in the memory (160) for executing the evaluation method is started immediately after the evaluation request has been generated.

3. Method according to claim 1 or 2, wherein the evaluation method comprises several different method parts (170) for evaluating different output data, wherein
- depending on the predetermined event different types of evaluation requests are generated and
- depending on the respective type of evaluation request generated one of the method parts (170) is started.

4. Method according to claim 3, wherein depending on the method part (170) which is started, access is made to output data stored in the database (70).

5. Method according to claim 3 or 4, wherein in case of a unique or repeated occurrence of predetermined events in connection with a particular evaluation object, respectively two or more evaluation requests are generated and one of the method parts (170) is started for only one of these evaluation requests if the other evaluation requests would start the same method part (170).

6. Method according to any of the preceding claims, wherein respectively one of the evaluation requests stored in the database (70) is selected and wherein depending on the evaluation request selected the evaluation program stored in the database (160) for executing the evaluation method is started and the evaluation method accesses to output data stored in the database (70) depending on the evaluation request.

7. Method according to claim 6, wherein the evaluation requests are stored in the database (70) in a specific order and respectively one of the evaluation requests stored in the database (70) is selected according to said order.

8. Method according to any of the preceding claims, wherein after a user having made a user request, the evaluation data generated are output, wherein the output data are generated before the user request.

9. Method according to any of the preceding claims, wherein the evaluation data generated are stored in the database (70).

10. Method according to any of the preceding claims, wherein at least one evaluation request is used for data exchange of the system with another system.

11. System (10) for generating evaluation data, wherein the system (10) is configured for executing the method according to any of the preceding claims.

12. System according to claim 11, wherein a computer network (20) is provided which comprises several computer devices (30, 40) which are connected among themselves and with the database (70) for exchanging data.

13. System according to claim 11 or 12, which is configured as a hospital information system (HIS, CIS).

14. Computer program for carrying out each of the steps of the method according to any of the claims 1 to 10 when executed on a data processing system.

## Revendications

1. Procédé destine à la production de données d'évaluation, dans lequel
- des données de sortie produites dans un hôpital dans des processus médicaux et/ou logistiques et/ou administratifs, sont mémorisées dans une base de données (70),
- des modifications des données de sortie mémorisées dans la base de données (70) sont contrôlées de façon continue par un déclencheur de base de données et dans le cas d'une modification de données de sortie, il est généré une requête d'évaluation pour évaluer des données de sortie, des données de sortie sont mémorisées ou modifiées dans la base de données (70) par un processus exécuté par un module de programme, une requête d'évaluation servant à l'évaluation de données de sortie étant générée simultanément et/ou une requête d'évaluation servant à l'évaluation de données de sortie étant générée lorsqu'un événement prédéfini se produit dans le sens où il est atteint un point temporel prédéfini ou une durée prédéfinie, ladite requête d'évaluation servant à l'évaluation de données de sortie étant mémorisées dans un tableau compris dans la base de données (70),
- il est contrôlé si une requête d'évaluation a été générée et a été mémorisée dans le tableau et dans le cas où le tableau comprend une requête d'évaluation, un programme d'évaluation mémorisé dans une mémoire (160) pour exécuter un procédé d'évaluation est démarré en fonction de la requête d'évaluation, et
- le procédé d'évaluation accède à des données de sortie mémorisées dans la base de données (70) en fonction de la requête d'évaluation et génère des données d'évaluation à partir des données de sortie, lesdites données d'évaluation comprenant des indicateurs clés de performance des processus médicaux et/ou logistiques et/ou administratifs dans l'hôpital.

2. Procédé selon la revendication 1, **caractérisé en ce que** la présence d'une requête d'évaluation est contrôlée de façon continue de façon à ce que le programme d'évaluation mémorisé dans la mémoire (160) et servant à exécuter le procédé d'évaluation soit démarré immédiatement après la génération de la requête d'évaluation.

3. Procédé selon la revendication 1 ou 2, **caractérisé en ce que** le procédé d'évaluation comprend plusieurs parties de procédé (170) différentes servant à évaluer différentes données de sortie, **caractérisé en ce que**
- en fonction de l'événement prédéfini, différents types de requêtes d'évaluation sont générés et
- en fonction du type respectif de requête d'évaluation générée, l'une des parties de procédé (170) est démarrée.

4. Procédé selon la revendication 3, **caractérisé en ce qu'**en fonction de la partie de procédé (170) démarrée, il est accédé à des données de sortie mémorisées dans la base de données (70).

5. Procédé selon la revendication 3 ou 4, **caractérisé en ce que** dans le cas où il se produit une seule fois des événements prédéfinis ou il se produit répétitivement des événements prédéfinis en rapport avec un objet d'évaluation particulier, respectivement deux ou plus de requêtes d'évaluation sont générées et l'une des parties de procédé (170) est démarrée pour une seule desdites requêtes d'évaluation si les autres requêtes d'évaluation provoqueraient le démarrage de la même partie de procédé (170).

6. Procédé selon l'une quelconque des revendications précédentes, **caractérisé en ce que** respectivement l'une des requêtes d'évaluation mémorisées dans la base de données (70) est sélectionnée et qu'en fonction de la requête d'évaluation sélectionnée, le programme d'évaluation mémorisé dans la base de données (160) er servant à exécuter le procédé d'évaluation est démarré et le procédé d'évaluation accède à des données de sortie mémorisées dans la base de données (70) en fonction de la requête d'évaluation.

7. Procédé selon la revendication 6, **caractérisé en ce que** les requêtes d'évaluation sont mémorisées dans la base de données (70) dans un ordre spécifique et que respectivement l'une des requêtes d'évaluation mémorisées dans la base de données (70) est sélectionnée en fonction dudit ordre.

8. Procédé selon l'une quelconque des revendications précédentes, **caractérisé en ce que** la requête d'utilisateur saisie par un utilisateur est suivie par la sortie des données d'évaluation générées, lesdites données de sortie étant générées avant la saisie de la requête d'utilisateur.

9. Procédé selon l'une quelconque des revendications précédentes, **caractérisé en ce que** les données d'évaluation générées sont mémorisées dans la base de données (70).

10. Procédé selon l'une quelconque des revendications précédentes, **caractérisé en ce qu'**au moins une requête d'évaluation sert à l'échange de données du système avec un autre système.

11. Système (10) destiné à produire des données d'évaluation, **caractérisé en ce que** le système (10) est conçu de façon à exécuter le procédé selon l'une quelconque des revendications précédentes.

12. Système selon la revendication 11, **caractérisé en ce qu'**il est prévu un réseau informatique (20) qui comprend plusieurs dispositifs informatiques (30, 40) connectés entre eux et avec la base de données (70) en vue de l'échange de données.

13. Système selon la revendication 11 ou 12 conçu comme un système d'information hospitalier (HIS, CIS).

14. Programme informatique servant à assurer, lorsqu'il est exécuté sur un système de traitement de données, l'application de chacune des étapes du procédé selon l'une quelconque des revendications 1 à 10.
